(19) Europäisches Patentamt
European Patent Office
Office européen des brevets

(11) EP 3 692 977 B1

(12) EUROPEAN PATENT SPECIFICATION

(45) Date of publication and mention of the grant of the patent:
**11.09.2024 Bulletin 2024/37**

(21) Application number: **19156152.1**

(22) Date of filing: **08.02.2019**

(51) International Patent Classification (IPC):
***A61K 8/34*** *(2006.01)* ***A61K 8/41*** *(2006.01)*
***A61Q 5/00*** *(2006.01)* ***A61K 8/891*** *(2006.01)*
***A61K 8/898*** *(2006.01)*

(52) Cooperative Patent Classification (CPC):
**A61K 8/416; A61K 8/342; A61K 8/891; A61K 8/898; A61Q 5/00**

# (54) COMPOSITION AND PROCESS FOR REDUCING THE DRYING TIME OF SOLID SUBSTRATES

ZUSAMMENSETZUNG UND VERFAHREN ZUR VERRINGERUNG DER TROCKNUNGSZEIT VON FESTEN SUBSTRATEN

COMPOSITION ET PROCÉDÉ POUR RÉDUIRE LE TEMPS DE SÉCHAGE DE SUBSTRATS SOLIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.08.2020 Bulletin 2020/33**

(73) Proprietor: **KAO CORPORATION**
**Tokyo 103-8210 (JP)**

(72) Inventors:
- **FUJINAGA, Hiroki**
  **Tokyo, 103-8210 (JP)**
- **TETSU, Makio**
  **Tokyo, 103-8210 (JP)**
- **PICKER, Andreas**
  **64297 Darmstadt (DE)**

(74) Representative: **Hoffmann Eitle**
**Patent- und Rechtsanwälte PartmbB**
**Arabellastraße 30**
**81925 München (DE)**

(56) References cited:

| | |
|---|---|
| **EP-A1- 3 168 251** | **WO-A1-00/00174** |
| **WO-A1-2013/082096** | **WO-A2-95/05800** |
| **DE-A1- 102015 222 976** | **DE-A1- 102015 223 028** |
| **FR-A1- 3 059 900** | **US-A1- 2007 141 007** |

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 692 977 B1

## Description

### Field of the invention

The present invention relates to a composition, method, and use to reduce the drying time of solid substrates.

### Background of the invention

People in developed countries and newly industrialized countries alike experience a large amount of pollution in their everyday life stemming from incinerating fossil fuels generating electrical energy, propelling cars and aircrafts, and heating or cooling their homes. In addition, many industrial manufacturing processes produce polluted solid products, which cannot easily be sold to customers or used in subsequent production processes without prior cleansing treatment. Thus, industry and consumers face problems of cleansing solid substrates to remove pollutants. As many cleansing procedures require the application of aqueous compositions, drying the respective substrate before further use is highly desired. However, if air-temperature is low, air humidity high, or a speedy drying of the substrate is required, heat drying is a common technique to accelerate the process. In this respect, heat drying requires the application of energy, and, thus, must be as short as possible to reduce environmental burden.

A prominent illustration of such burden to cosmetic customers and environment has been published in a survey with more than 200 women and 200 teenagers over age 16 conducted in 2014 in the United States showing that women spend an average of 55 minutes each morning on their appearance [[1] https://www.today.com/health/how-we-did-today-aol-ideal-real-body-image-survey-[2] https://www.today.com/health/stop-obsessing-women-spend-2-weeks-year-their-appearance-today-2D12104866]. A not negligible part of this time is spent on the drying procedure of hair, especially for women with long hair. In light of this background it is not surprising that the cosmetic industry in general and hair care industry in particular attempts to find solutions to help long-hair customers accelerating their daily procedure and, thus, to save time. Most of the available products are so-called blow dry leave in sprays, based on lower alcohols solutions that help to evaporate water during the blow dry process. Some very few products are available in the rinse off area, promising reduced blow dry time and facilitating natural, non-alcohol-based evaporation. Unfortunately, these products are typically based on high concentrations of cyclopentasiloxanes (e.g. Mintel # 2303144), a silicone compound that quickly evaporates. However, this raw material is under regulatory discussion in some regions of the world.

When investigating the drying process of substrates such as hair, it starts prior to blow drying evaporation, in particular immediately after the final rinsing step of hair. The very first step is the drainage of water from the substrate. Moreover, this step is the decisive step for removing most of the water from the substrate. Thus, the more efficient the drainage in the drying process is prior to applying heat, the less time the customer/industry spends on later stage heat drying and the less energy has to be applied for drying.

In summary, it is paramount to research products that help to increase the velocity of water outflow for the convenience of consumers, efficiency of industry, and protection of the environment.

Inventors of the present invention have unexpectedly found out that a composition comprising certain dialkyl quaternary ammonium cationic surfactants, low molecular weight dimethylpolysiloxanes, aminated silicones, and fatty alcohols reduces the drying time of solid substrates, in particular of keratin fibers, enhance drainage of water from substrates, provide for a dry appearance, good bundle separation of keratin fibers, and good care feeling. By the present invention it is aimed to balance a fast water drainage with a good care feeling of the substrates.

Products comprising bis-(isostearoyl/oleoyl isopropyl) dimonium methosulfate and dimethicone are known from the prior art (e.g. Mintel # 5534485, # 3904857, # 5306377). However, the present invention utilizes low molecular weight dimethicones and certain weight ratios and concentrations of the compound, and, thus, the invention is novel.

FR3059900 discloses bis-(isostearoyl/oleoyl isopropyl) dimonium methosulfate and amodimethicone, but the document is silent on low molecular weight dimethicones, and, thus, the invention is novel.

EP3168251 discloses bis-(isostearoyl/oleoyl isopropyl) dimonium methosulfate and dimethicone having a viscosity of 100 cs, and, therefore, a higher molecular weight as the dimethicones required by the present invention. Furthermore, it does not achieve the technical effect of the present invention as shown in the comparative examples.

DE102015222976 discloses bis-(isostearoyl/oleoyl isopropyl) dimonium methosulfate and high viscosity dimethicone, and, therefore, a higher molecular weight as the dimethicones required by the present invention. Furthermore, it does not achieve the technical effect of the present invention. WO 95/05800 discloses topical personal care compositions comprising a polysiloxane-grafted adhesive polymer and a drying aid.

Despite the numerous attempts of the prior art, none of disclosures solve the problem of the present invention.

## Summary of the invention

Thus, the first object of the present invention is a composition for reducing the drying time of solid substrates, preferably of keratin substrates, more preferably of human keratin substrates, further more preferably of human hair comprising:

a) One or more quaternary ammonium surfactant(s) according to the following general structure

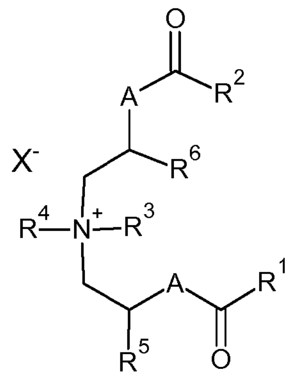

with the provision that $R^1$ and $R^2$ are $C_{12}$-$C_{21}$ linear or branched, saturated or unsaturated hydrocarbon groups, which may be the same or different and may be optionally substituted with one or more substituents selected from halogen, hydroxyl, amino, and $C_1$-$C_4$ alkoxy,

$R^3$ and $R^4$ are $C_1$-$C_6$ linear or branched, saturated or unsaturated hydrocarbon groups, which may be the same or different and may be optionally substituted with one or more substituents selected from halogen, hydroxyl, amino, and

$R^5$ and $R^6$ are H, $C_1$-$C_{12}$ linear or branched, saturated or unsaturated hydrocarbon groups, which may be the same or different and the hydrocarbon groups may be optionally substituted with one or more substituents selected from halogen, hydroxyl, amino, and $C_1$-$C_4$ alkoxy, and

A is selected from O and N, and X- is selected from $Cl^-$, Br, $I^-$, sulfate, and methosulfate,

b) one or more dimethylpolysiloxane(s) having a weight average molecular weight $M_w$ in the range of 190 g/mol to 6000 g/mol determined by gel permeation chromatography,

c) one or more aminated silicone,

d) one or more fatty alcohol having a saturated or unsaturated, branched or linear $C_{12}$ to $C_{22}$ alkyl chain,

wherein the total concentration of compounds according to a) is in the range of 0.1 % to less than 5% by weight, calculated to the total weight of the composition, and

wherein the weight ratio of the compounds a) to (b) + c)) is in the range of 0.075 to 8.

The second object of the present invention is a method for reducing the drying time of solid substrates, preferably of keratin substrates, more preferably of human keratin substrates, further more preferably of human hair comprising the steps of:

a) contacting the solid substrate with an aqueous composition,

b) applying the composition according to any of the claims 1 to 12 onto the substrate,

c) leaving the composition onto the substrate for a time period of 10 s to 600 s,

d) rinsing-off the solid substrate with water,

e) leaving the solid substrate for allowing drainage of water,

f) optionally drying the substrate with a heating device delivering an air temperature in the range of 35°C to 230°C.

It is preferred from the viewpoint of convenience for the user that between steps d) and f) no towel drying is performed.

However, in another aspect, it is preferred from the viewpoint of drying efficiency, time saving, and energy saving that towel drying is performed between steps d) and f).

It is further preferred from the viewpoint of user experience and routine that the aqueous composition of step a) is an aqueous conditioning composition.

The third object of the present invention is a use of a composition as defined above for enhancing water drainage from solid substrates.

**Detailed description of the invention**

The composition is suitable to reduce the drying time on various solid substrates such as wood, concrete, plastic surfaces, metal surfaces, and fiber surfaces. Suitable fiber surfaces are cotton, keratin fibers such as wool and hair. A suitable solid keratin substrate also is skin, preferably human skin. However, the present invention is particularly suitable for human keratin fibers, preferably human hair, from the viewpoint of its safety for human application.

**Compounds according to a)**

Suitable compounds according to a) falling into the ambit of the structure according to claim 1 are displayed in Table 1.

Table 1: Suitable compounds according to a):

| Trade name | CAS/INCI | $R^1$ | $R^2$ | $R^3$ | $R^4$ | $R^5$ | $R^6$ | A |
|---|---|---|---|---|---|---|---|---|
| Varisoft EQ100* | Quaternium 98 | Iso-stearoyl | Oleoyl | $CH_3$ | $CH_3$ | $CH_3$ | $CH_3$ | O |
| Tetranyl CO-40** | 97338-05-1 | $C_{18}$ alkenyl | $C_{18}$ alkenyl | $CH_3$ | $CH_2CH_2OH$ | H | H | O |
| n.a. | 32208-04-1 | $C_{18}$ alkyl | $C_{18}$ alkyl | $CH_3$ | $CH_2CH_2OH$ | H | H | O |
| n.a. | 161294-46-8 | $C_{16}$ alkyl | $C_{16}$ alkyl | $CH_3$ | $CH_2CH_2OH$ | H | H | O |
| n.a. | 1862231-90-0 | $C_{16}$ alkyl | $C_{16}$ alkyl | $CH_3$ | $CH_3$ | $CH_3$ | $CH_3$ | O |
| n.a. | 32208-07-4 | $C_{18}$ alkyl | $C_{18}$ alkyl | $CH_3$ | $CH_3$ | $CH_3$ | $CH_3$ | O |
| n.a. | 112065-30-2 | $C_{18}$ alkenyl | $C_{18}$ alkenyl | $CH_3$ | $CH_3$ | $CH_3$ | $CH_3$ | O |
| Varisoft 222LT90* | 92888-37-4 | $C_{18}$ alkenyl | $C_{18}$ alkenyl | $CH_3$ | $CH_2CH_2OH$ | H | H | N |
| * Evonik Nutrition and Care GmbH<br>**Kao Corp. | | | | | | | | |

It is preferred from the viewpoint of biodegradability that the compound according to

a) has the following general structure

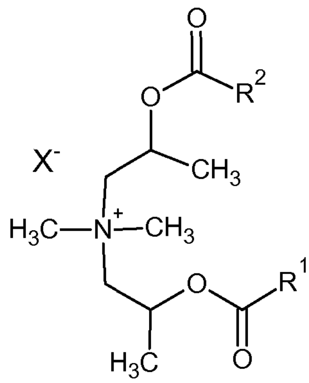

with the provision that $R^1$ and $R^2$ are $C_{11}$-$C_{21}$ linear or branched, saturated or unsaturated hydrocarbon groups, which may be the same or different and may be optionally substituted with one or more substituents selected from halogen, hydroxyl, amino, and $C_1$-$C_4$ alkoxy, and $X^-$ is selected from $Cl^-$, Br, $I^-$, sulfate, and methosulfate.

The most preferred compound according to a) is bis-(isostearoyl/oleoyl isopropyl) dimonium and/or its salt(s), from the viewpoint of commercial availability as well as biodegradability. Its suitable salts are $Cl^-$, $Br^-$, $I^-$, sulfate, and methosulfate. The most preferred salt is methosulfate from the viewpoint of commercial availability. The compound is commercially available under the trade name Varisoft EQ100 from Evonik Nutrition and Care GmbH ad has an INCI name application of Quaternium 98.

The total concentration of compounds according to a) is 0.1% by weight or more, preferably 0.2% by weight or more, more 0.5% by weight or more, calculated to the total weight of the composition, from the viewpoint of water drainage, appearance and bundle separation.

The total concentration of compounds according to a) is less than 5% by weight, preferably 4% by weight or less, further more preferably 3% by weight or less, still further more preferably 2% by weight or less, calculated to the total weight of the composition, from the viewpoint of water drainage, appearance and bundle separation.

For attaining the above-mentioned effects, the total concentration of compounds according to a) is in the range of 0.1% by weight to less than 5% by weight, preferably in the range of 0.2% by weight to 4% by weight, more preferably in the range of 0.5% by weight to 3% by weight, still more preferably in the range of 0.5% by weight to 2% by weight, calculated to the total of the composition.

**Compounds according to b)**

For compounds according to b), weight average molecular weight needs to be determined. Molecular weight determination for measuring $M_w$ according to the present invention is suitably conducted by gel permeation chromatography.

The weight average molecular weight Mw of compound b) is determined by the method described below.

A sample is dissolved in chloroform at 25°C, so as to have a concentration of 0.5 g/100 mL. Using the following measurement apparatus and analytical column, chloroform is allowed to flow as an eluent at a rate of 1.0 ml per minute, and the column is stabilized in a thermostat at 40°C. One-hundred microliters of the sample solution is injected to the column to take the measurements. The detectors for detecting the compounds during chromatography are refractive index detectors The molecular weight of the sample is calculated on the basis of a calibration curve previously prepared. As the calibration curve of the molecular weight, one prepared by using several kinds of monodisperse polystyrenes as a standard sample is used.

Measurement Apparatus: HLC-8320GPC commercially available from Tosoh Corporation.

Analytical Column: K-804L + K-804L commercially available from SHOWA DENKO K.K.

Thus, the weight average molecular weight $M_w$ of compound b) is preferably 190 g/mol or more, more preferably 260 g/mol or more, further more preferably 320 mol/g or more, determined by gel permeation chromatography, from the viewpoint of water drainage and appearance.

The weight average molecular weight $M_w$ of compound b) is preferably 6000 g/mol or less, more preferably 5200 g/mol or less, further more preferably 2300 mol/g or less, still further more preferably 1300 mol/g or less, still further more preferably 760 mol/g or less, determined by gel permeation chromatography, from the viewpoint of water drainage, appearance and bundle separation.

For attaining the above-mentioned effects, the weight average molecular weight $M_w$ of compound b) is in the range of 190 g/mol to 6000 g/mol, more preferably in the range of 190 g/mol to 5200 g/mol, further more preferably in

the range of 190 g/mol to 2300 g/mol, still further more preferably in the range of 260 g/mol to 1300 g/mol, still further more preferably in the range of 320 g/mol to 760 g/mol, determined by gel permeation chromatography.

The viscosity at 25°C of compound b) is preferably 0.65 $mm^2/s$ or more, more preferably 1 $mm^2/s$ or more, further more preferably 1.5 $mm^2/s$ or more, from the viewpoint of water drainage and appearance.

The viscosity at 25°C of compound b) is preferably 60 $mm^2/s$ or less, more preferably 50 $mm^2/s$ or less, further more preferably 20 $mm^2/s$, still further more preferably 10 $mm^2/s$ or less, still further more preferably 5 $mm^2/s$ or less, from the viewpoint of water drainage, appearance and bundle separation.

For attaining the above-mentioned effects, the viscosity at 25°C of compound b) is in the range of 0.65 $mm^2/s$ to 60 $mm^2/s$, more preferably in the range of 0.65 $mm^2/s$ to 50 $mm^2/s$, further more preferably in the range of 0.65 $mm^2/s$ to 20 $mm^2/s$ still further more preferably in the range of 1 $mm^2/s$ to 10 $mm^2/s$, still further more preferably in the range of 1.5 $mm^2/s$ to 5 $mm^2/s$.

The viscosity at 25°C of compound b) is measured with an Ubbelohde viscometer according to ASTM D 445-46 T or JIS Z 8803.

It is preferred that the total concentration of compounds according to b) is 0.1% by weight or more, preferably 0.2% by weight or more, further more preferably 0.5% by weight or more, still further more preferably 1.0% by weight or more, calculated to the total weight of the composition, from the viewpoint of water drainage, appearance, and bundle separation.

It is preferred that the total concentration of compounds according to b) is 10% by weight or less, preferably more preferably 7.5% by weight or less, further more preferably 5% by weight or less, still further more preferably 2.5% by weight or less calculated to the total weight of the composition, from the viewpoint of care feeling, water drainage, and cost of goods aspect.

For attaining the above mentioned effects, the total concentration of compounds according to b) is in the range of 0.1% to 10% by weight, preferably in the range of 0.2% to 7.5% by weight, more preferably in the range of 0.5% to 5% by weight, further more preferably in the range of 0.5% to 2.5% by weight, still further more preferably in the range of 1.0% to 2.5% by weight, calculated to the total weight of the composition.

Furthermore, the preferable weight ratio of the compounds a) to (b) + c)) is in the range of 0.15 to 3, preferably in the range of 0.3 to 2, from the viewpoint of water drainage and appearance.

**Compounds according to c)**

The composition of the present invention comprise one or more aminated silicone. Such aminated silicones are known under their CTFA name amodimethicone.

Suitable aminated silicones may have a primary, secondary, tertiary amino group and/or a quaternary ammonium group.

Such aminated silicones are available as liquid composition with almost 100% active matter content (fluids), or as emulsion. Macro- and microemulsions are available.

The term microemulsion within the meaning of the present invention is to be understood as an emulsion with a droplet size in the range of 50 nm to 1000 nm. Macroemulsions have droplet sizes of more than 1000 nm.

Suitable aminated silicone fluids are, for example, bis-hydroxy/methoxy amodimethicone available under the trade name DC-AP-8087 fluid from Dow Corning Corp.

Suitable aminated silicone macroemulsions are, for example, dimethoxysilyl ethylenediaminopropyl dimethicone macroemulsion available under the trade name Belsil ADM 6102E from Wacker Chemie AG, or DC 969 available from Dow Corning Corp.

Suitable aminosilicone microemulsions are offered by Wacker Corp. in particular Wacker Belsil ADM 6057 and Wacker Belsil ADM 8020 VP. Further suitable microemulsions are dimethoxysilyl ethylenediaminopropyl dimethicone microemulsions available under the trade name Xiameter MEM 8194 by Xiameter Corp., silicone quaternium 16 microemulsion available under the trade name DC CE 5-7133 from Dow Corning Corp., and silicone quaternium 18 microemulsion available under the trade name Silsoft Q PMF. Further suitable microemulsions of aminated silicones are offered by Shin-Etsu Corp. under the trade name X-52-2265, and DC CE 8170 AF by Dow Corning Corp.

Suitable copolymers or crosspolymers of aminated silicones are, for example, bisisobutyl PEG/PPG-20/35/amodimethicone copolymer available under the trade name DC CE 8401 from Dow Corning Corp., silicone quaternium-16/glycidoxy dimethicone crosspolymer available under the trade name DC CE 7081 from Dow Corning Corp.

It is preferred from the viewpoint of easy formulation preparation, that the compound(s) according to c) is/are aqueous emulsions of aminated silicones.

The preferred aminated silicones are aminated silicone macro- and microemulsions. The most preferred aminated silicone is dimethoxysilyl ethylenediaminopropyl dimethicone macroemulsion available under the trade name Belsil ADM 6102E from Wacker Chemie AG, from the viewpoint of enhancing care feeling to a sufficient degree and maintaining a quick drying property of the substrate.

It is preferred that the total concentration of compounds according to c) is 0.05% by weight or more, more preferably 0.1% by weight or more, further more preferably 0.25% by weight or more, calculated to the total weight of the composition, from the viewpoint of enhancing care feeling to a sufficient degree.
It is preferred that the total concentration of compounds according to c) is 5% by weight or less, more preferably 2% by weight or less, further more preferably 1.5% by weight or less, calculated to the total weight of the composition, from the viewpoint of enhancing water drainage and appearance.
For attaining the above-mentioned effects, it is preferred that the total concentration of compounds according to c) is in the range of 0.05% to 5% by weight, more preferably in the range of 0.1% to 2% by weight, further more preferably in the range of 0.25% to 1.5% by weight, calculated to the total weight of the composition.
For maintaining good water drainage and good care feeling, it is preferred that the weight ratio of compound b) to c) is in the range of 5 to 0.2.

**Compounds according to d)**

The composition of the present invention comprises one or more fatty alcohol having a saturated or unsaturated, branched or linear $C_{12}$ to $C_{22}$ alkyl chain as compound d).
Suitable fatty alcohols having branched or linear, saturated or unsaturated $C_{12}$ to $C_{22}$ alkyl chains are, for example, lauryl alcohol, tridecyl alcohol, myristyl alcohol, pentadecyl alcohol, cetyl alcohol, palmitoleyl alcohol, heptadecyl alcohol, stearyl alcohol, oleyl alcohol, nonadecyl alcohol, arachidyl alcohol, behenyl alcohol, and/or their mixtures.
It is preferred that one or more fatty alcohol is selected from fatty alcohols having branched or linear, saturated or unsaturated $C_{14}$ to $C_{18}$ alkyl chains, preferably saturated $C_{14}$ to $C_{18}$ alkyl chains, and/or their mixtures, more preferably it is a mixture of fatty alcohols having saturated and linear $C_{14}$ and $C_{16}$ alkyl chains, from the viewpoint of enhancing care feeling on keratin fibers, and good compatibility with hair conditioning products.
The preferred fatty alcohols are myristyl alcohol, cetyl alcohol, and stearyl alcohol, from the viewpoint of enhancing care feeling on keratin fibers, and good compatibility with hair conditioning products.
Preferably, the total concentration of compounds according to d) is 0.2% by weight or more, more preferably 0.5% by weight or more, further more preferably 1% by weight or more, still further more preferably 2% by weight or more, calculated to the total weight of the composition, from the viewpoint of enhancing care feeling and appearance.
Preferably, the total concentration of compounds according to d) is 10% by weight or less, more preferably 8% by weight less, further more preferably 6% by weight or less, calculated to the total weight of the composition, from the viewpoint of preparing a stable emulsion.
For attaining the above-mentioned effect, preferably the total concentration of compounds according to d) is in the range of 0.2% to 10% by weight, preferably in the range of 0.5% to 8% by weight, further more preferably in the range of 1% to 6% by weight, still further more preferably in the range of 2% to 6% by weight, calculated to the total weight of the composition.

**Other lipophilic compounds according to e)**

Optionally, the composition of the present invention may comprise other lipophilic compounds as compound e) different from compounds b), c) and d).
Suitable compounds e) are esters of alcohols having a linear or branched $C_3$ to $C_{18}$ alkyl chain with fatty acids having a linear or branched, saturated or unsaturated $C_{12}$ to $C_{22}$ alkyl chain are isopropyl palmitate, isopropyl myristate, octyl palmitate, isocetyl palmitate, octyl stearate, oleyl oleate, ethylhexyl hydroxystearate, myristyl myristate, behenyl behenate, and/or their mixtures.
Further compounds e) are vegetable oils such as jojoba oil, avocado oil, sunflower seed oil, walnut oil, peanut oil, olive oil, rapeseed oil, cottonseed oil, palm oil, sesame oil, soybean oil, coconut oil, safflower oil, almond oil, macadamia nut oil, grapefruit seed oil, lemon kernel oil, orange kernel oil, apricot kernel oil, castor oil, argan oil, tamanu oil, and/or their mixtures.
Further suitable compounds e) are petrolatum-based products are liquid paraffins, especially paraffinum perliquidum and paraffinum subliquidum, and mineral oil, in particular white mineral oil, and/or their mixtures.
It is preferred that the total concentration of compounds according to e) is 0.1% by weight or more, more preferably 0.5% by weight or more, further more preferably 0.75% by weight or more, calculated to the total weight of the composition, from the viewpoint of providing sufficient conditioning effect to keratin fibers.
It is preferred that the total concentration of compounds according to e) is 10% by weight or less, more preferably 8% by weight or less, further more preferably 6% by weight or less, calculated to the total weight of the composition, from the viewpoint of cost of goods and stability of an aqueous composition.
Suitably, for attaining the above-mentioned effects, the total concentration of compounds according to e) is in the range of 0.1% to 10% by weight, preferably 0.5% to 8% by weight, more preferably 0.75% to 6% by weight, calculated

to the total weight of the composition.

It is preferred within this aspect of the present invention that the composition is an emulsion from the viewpoint of stability of the composition.

**Other surfactants different from a)**

For formulating an emulsion and from the viewpoint of further enhancing the stability of the composition, it may comprise one or more surfactant(s) different from a), preferably selected from cationic surfactants and/or non-ionic surfactants.

It is preferred from the viewpoint of enhancing composition stability and/or enhancing conditioning effect to keratin fibers that the total concentration of surfactants different from a) is in the range of 0.1% to 10% by weight, preferably in the range of 0.25% to 6% by weight, calculated to the total weight of the composition.

It is further preferred from the viewpoint of cosmetic acceptance of the composition that the pH of the composition is in the range of 3 to 8, preferably in the range of 3.5 to 7, more preferably in the range of 3.5 to 4.5, from the viewpoint of user application in a conditioning composition.

The following examples are to illustrate the present invention, but not to limit it.

**EXAMPLES**

| | | | Working Example | | | | | Comparative Example | | |
|---|---|---|---|---|---|---|---|---|---|---|
| | | | 1 | 2 | 3 | 4 | 5 | 1 | 2 | 3 |
| **Composition** | Component (a) | Quaternium-98 *1 | 1.5 | 1.5 | 1.5 | 1.5 | 1.5 | 1.5 | 1.5 | 1.5 |
| | Component (b) | DMPS (2 $mm^2/s$, Mw 392)*2 | 2.25 | 2.0 | 0.75 | 2.25 | 2.25 | - | 2.5 | - |
| | | DMPS (100 $mm^2/s$, Mw 8870)*3 | - | - | - | - | - | - | - | 2.25 |
| | Component (c) | Amodimethicone*4 | 0.25 | 0.50 | 0.75 | 0.25 | 0.25 | - | - | 0.25 |
| | Component (d) | Fatty alcohol ($C_{14}/_{16}$=2/3)*5 | 5.0 | 5.0 | 5.0 | - | - | - | 5.0 | 5.0 |
| | | Fatty alcohol ($C_{16}/_{18}$=2/3)*6 | - | - | - | 5.0 | - | - | - | - |
| | | Fatty alcohol ($C_{18}$)*7 | - | - | - | - | 5.0 | - | - | - |
| | | NaOH/HCl | q.s. ad pH 3.7 ± 0.5 | | | | | | | |
| | | Water | Ad 100.0 | | | | | | | |
| | Weight ratio a) to (b) + c)) | | 0.6 | 0.6 | 1 | 0.6 | 0.6 | | 0.6 | 0.6 |
| **Evaluation** | Water Drainage (%) | | 52.4 | 52.8 | 51.1 | 52.0 | 51.3 | 41.9 | 51.5 | 48.1 |
| | Appearance | | 11.6 | 12.6 | 12.8 | 11.9 | 12.1 | 14.4 | 9.2 | 14.1 |
| | Bundle Separation | | 8 | 8 | 8 | 7 | 7 | 5 | 8 | 5 |
| | Care feeling | | 4.3 | 4.7 | 5 | 4.3 | 4.7 | 2.3 | 3 | 4.7 |

*1 Varisoft EQ100 obtained from Evonik Nutrition and Care GmbH
*2, 3 KF-96 2 cs and 100 cs series obtained from Shin-Etsu Chemical Co. Ltd., DMPS = dimethylpolysiloxane
*4 Belsil ADM 6102E obtained from Wacker Chemie AG (40% active)
* 5 KALCOL 4098/KALCOL 6098 obtained from Kao Corp., mixing weight ratio 2 parts KALCOL 4098, 1 part KALCOL 6098
* 6 KALCOL 6098/KALCOL 8098 obtained from Kao Corp., mixing weight ratio 2 parts KALCOL 6098, 1 part KALCOL 6098
* 7 KALCOL 8098 obtained from Kao Corp.

## Methods

### Preparation of hair streaks for water drainage measurements

Human hair fibers (Caucasian, 15 cm long) were purchased from Fischbach and Miller Haar, Laupheim, Germany. Hair fibers were merged to yield about 10 g hair per streak. The merged hair streaks were bleached twice with a bleaching solution comprising 6% by weight ammonia (25% active) and 2% by weight ammonium chloride having a pH of 9.8, and 6% by weight hydrogen peroxide (50% active). The hair streaks were fully immersed with the bleaching solution in a water bath for 30 min per bleaching session. After completing the 2 bleaching treatments, the streaks were rinsed with water for 2 min, combed, and allowed to air-dry.

### Water drainage measurements

As a first step, dry weights of the merged hair streaks were determined. For this purpose, each streak was allowed to be wetted with water in a beaker for 15 min, then they were shampooed with a commercial shampoo available under the brand name Goldwell Dualsenses Scalp Specialist Deep Cleansing Shampoo, rinsed with lukewarm water for 1.5 min, and allowed to completely air-dry. Dry weights ($M_0$) were recorded.

For determination of wet weight ($M_1$) the hair streaks were allowed to be wetted for 12 min in a shaking bath filled with water. 5 g of the composition of the examples above was applied to each merged hair streak, the streaks were massaged for 1 min and the composition was allowed to rest for 2 min. The compositions were then rinsed off with lukewarm water for 1 min and water was allowed to drain for 2 min. Hair streaks were then centrifuged for 5 min 30 s (70 rpm, ~ 0.44 g) and after centrifugation the wet weights ($M_1$) of the streaks were determined. Water drainage (%) was then calculated according to the following equation:

$$\mathit{Water\ drainage}\ (\%) = \left(1 - \frac{M_1 - M_0}{M_0}\right) \cdot 100\%$$

### Appearance measurements

Caucasian blond hair streaks were obtained from volunteers and merged as described above, but not bleached. The streaks were the shampooed with a commercially available shampoo under the brand name Goldwell Dualsenses Scalp Specialist Deep Cleansing Shampoo, thoroughly rinsed, and allowed to air-dry completely. After complete air-drying of the hair streaks, dry hair color was measured by spectrophotometrical analysis with a Datacolor 45G CT instrument obtained from Datacolor Inc., Lawrenceville, NJ, USA. 5 measurements points on the hair streaks were averaged ($L_1$, $a_1$, $b_1$).

Hair streaks were then washed again with a commercially available shampoo under the brand name Goldwell Scalp Specialist Deep Cleansing Shampoo and rinsed-off with water. Then 3 g of the example compositions from above were applied onto the hair streaks, massaged for 1 min, and allowed to rest for 1 min. The streaks were then rinsed with lukewarm water for 1 min and water was drained by squeezing the hair streaks with the operator's finger. Then the streaks were blotted dry with a towel applying constant pressure onto the hair streaks (under use of a defined weight) for 30 s. The hair streaks were then combed through and color measurements of wet streaks were conducted at 5 different positions on the streak ($L_2$, $a_2$, $b_2$)

Based on the CIE*Lab color space results obtained by the measurements, $\Delta\ E_{ab}$ values for color difference were calculated according to the following equation:

$$\Delta\, E_{ab} = \sqrt{(L_2 - L_1)^2 + (a_2 - a_1)^2 + (b_2 - b_1)^2}$$

### Bundle separation

Hair streaks were prepared as for appearance measurements. After complete air-drying, the streaks were immersed with 3 g of the example compositions, thoroughly rinsed with lukewarm water, and towel-dried. Bundle separation was then evaluated independently by 3 experts without providing details of the composition to the experts prior to their evaluation. The experts were asked to visually judge and rate the bundle separation according to the following criteria:

3: completely separated (many small bundles)

2: partially separated (some big and small bundles)
1: little separation (many big bundles)

The judgements of the experts were then added to yield a total score and are reported as integer numbers.

**Care feeling**

The hair streaks were prepared as laid out for appearance measurements and bundle separation. After complete air-drying, the hair streaks were wetted with lukewarm tap water and immersed with 3 g of the example compositions, thoroughly rinsed with lukewarm water, and towel-dried. Care feeling was then evaluated independently by 3 experts without providing details of the composition to the experts prior to their evaluation. The experts were asked to touch and feel the hair streaks and to rate the care feeling according to the following criteria:

5: Very smooth and soft feeling
4: Good smooth and soft feeling
3: Acceptable smooth and soft feeling
2: Low smooth and soft feeling
1: No smoothness and rough feeling.

The ratings of the experts were then added and averaged to yield an average care feeling score.

## Claims

**1.** A composition for reducing the drying time of solid substrates, preferably of keratin substrates, more preferably of human keratin substrates, further more preferably of human hair comprising:

a) One or more quaternary ammonium surfactant(s) according to the following general structure

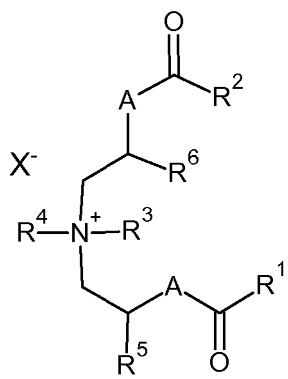

with the provision that $R^1$ and $R^2$ are $C_{12}$-$C_{21}$ linear or branched, saturated or unsaturated hydrocarbon groups, which may be the same or different and may be optionally substituted with one or more substituents selected from halogen, hydroxyl, amino, and $C_1$-$C_4$ alkoxy,
$R^3$ and $R^4$ are $C_1$-$C_6$ linear or branched, saturated or unsaturated hydrocarbon groups, which may be the same or different and may be optionally substituted with one or more substituents selected from halogen, hydroxyl, amino, and
$R^5$ and $R^6$ are H, $C_1$-$C_{12}$ linear or branched, saturated or unsaturated hydrocarbon groups, which may be the same or different and the hydrocarbon groups may be optionally substituted with one or more substituents selected from halogen, hydroxyl, amino, and $C_1$-$C_4$ alkoxy, and
A is selected from O and N, and $X^-$ is selected from $Cl^-$, Br, $I^-$, sulfate, and methosulfate,

b) one or more dimethylpolysiloxane(s) having a weight average molecular weight $M_w$ in the range of 190 g/mol to 6000 g/mol determined by gel permeation chromatography,
c) one or more aminated silicone,
d) one or more fatty alcohol having a saturated or unsaturated, branched or linear $C_{12}$ to $C_{22}$ alkyl chain,
wherein the total concentration of compounds according to a) is in the range of 0.1% to less than 5% by weight,

calculated to the total weight of the composition, and
wherein the weight ratio of the compounds a) to (b) + c)) is in the range of 0.075 to 8.

2. The composition according to claim 1 **characterized in that** the total concentration of compounds according to b) is in the range of 0.1% to 10% by weight, preferably in the range of 0.2% to 7.5% by weight, more preferably in the range of 0.5% to 5% by weight, further more preferably in the range of 0.5% to 2.5% by weight, still further more preferably in the range of 1% to 2.5% by weight, calculated to the total weight of the composition.

3. The composition according to claims 1 and/or 2 **characterized in that** the weight ratio of the compounds a) to (b) + c)) is in the range of 0.15 to 3, preferably in the range of 0.3 to 2.

4. The composition according to any of the preceding claims **characterized in that** the compound according to a) has the following general structure

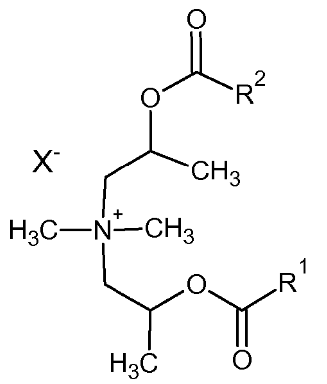

with the provision that $R^1$ and $R^2$ are $C_{11}$-$C_{21}$ linear or branched, saturated or unsaturated hydrocarbon groups, which may be the same or different and may be optionally substituted with one or more substituents selected from halogen, hydroxyl, amino, and $C_1$-$C_4$ alkoxy, and $X^-$ is selected from $Cl^-$, $Br$, $I^-$, sulfate, and methosulfate.

5. The composition according to any of the preceding claims **characterized in that** the compound according to a) is bis-(isostearoyl/oleoyl isopropyl) dimonium and/or its salt(s).

6. The composition according to any of the preceding claims **characterized in that** the total concentration of compounds according to d) is in the range of 0.2% to 10% by weight, preferably in the range of 0.5% to 8% by weight, further more preferably in the range of 1% to 6% by weight, still further more preferably in the range of 2% to 6% by weight, calculated to the total weight of the composition.

7. The composition according to any of the preceding claims **characterized in that** the compounds according to d) are selected from myristyl alcohol, cetyl alcohol, and stearyl alcohol, and/or their mixtures.

8. The composition according to any of the preceding claims **characterized in that** the total concentration of compounds according to c) is in the range of 0.05% to 5% by weight, more preferably in the range of 0.1% to 2% by weight, further more preferably in the range of 0.25% to 1.5% by weight, calculated to the total weight of the composition.

9. The composition according to any of the preceding claims **characterized in that** the weight ratio of compound b) to c) is in the range of 5 to 0.2.

10. The composition according to any of the preceding claims **characterized in that** the compound(s) according to c) is/are aqueous emulsions of aminated silicones.

11. The composition according to any of the preceding claims **characterized in that** the composition comprises one or more surfactant(s) different from a), preferably selected from cationic surfactants and/or non-ionic surfactants.

12. The composition according to any of the preceding claims **characterized in that** the pH of the composition is in the range of 3 to 8, preferably in the range of 3.5 to 7, more preferably in the range of 3.5 to 4.5.

13. A method for reducing the drying time of solid substrates, preferably of keratin substrates, more preferably of human keratin substrates, further more preferably of human hair comprising the steps of:

a) contacting the solid substrate with an aqueous composition,
b) applying the composition according to any of the claims 1 to 12 onto the substrate,
c) leaving the composition onto the substrate for a time period of 10 s to 600 s,
d) rinsing-off the solid substrate with water,
e) leaving the solid substrate for allowing drainage of water,
f) optionally drying the substrate with a heating device delivering an air temperature in the range of 35°C to 230°C.

14. The method according to claim 13 **characterized in that** between steps d) and f) no towel drying is performed.

15. Use of a composition as defined in the claims 1 to 12 for enhancing water drainage from solid substrates.

**Patentansprüche**

1. Zusammensetzung zur Verringerung der Trocknungszeit von festen Substraten, vorzugsweise von Keratinsubstraten, besonders bevorzugt von menschlichen Keratinsubstraten, ferner besonders bevorzugt von menschlichem Haar, umfassend:

a) ein oder mehrere quaternäre(s) Ammonium-Tensid(e) gemäß der folgenden allgemeinen Struktur

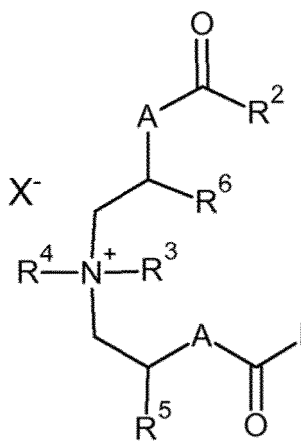

mit der Maßgabe, dass $R^1$ und $R^2$ sind $C_{12}$-$C_{21}$ lineare oder verzweigte, gesättigte oder ungesättigte Kohlenwasserstoffgruppen, die gleich oder verschieden sein können und gegebenenfalls mit einem oder mehreren Substituenten, ausgewählt aus Halogen, Hydroxyl, Amino und $C_1$-$C_4$-Alkoxy, substituiert sein können,
$R^3$ und $R^4$ sind $C_1$-$C_6$ lineare oder verzweigte, gesättigte oder ungesättigte Kohlenwasserstoffgruppen, die gleich oder verschieden sein können und gegebenenfalls mit einem oder mehreren Substituenten, ausgewählt aus Halogen, Hydroxyl, Amino, substituiert sein können, und
$R^5$ und $R^6$ sind H, $C_1$-$C_{12}$ lineare oder verzweigte, gesättigte oder ungesättigte Kohlenwasserstoffgruppen, die gleich oder verschieden sein können und die Kohlenwasserstoffgruppen können gegebenenfalls mit einem oder mehreren Substituenten, ausgewählt aus Halogen, Hydroxyl, Amino und $C_1$-$C_4$-Alkoxy, substituiert sein, und
A ist ausgewählt aus O und N, und $X^-$ ist ausgewählt aus $Cl^-$, $Br^-$, $I^-$, Sulfat und Methosulfat,

b) ein oder mehrere Dimethylpolysiloxan(e) mit einem Gewichtsmittel des Molekulargewichts $M_w$ im Bereich von 190 g/mol bis 6000 g/mol, bestimmt durch Gelpermeationschromatographie,
c) ein oder mehrere aminierte Silikone,
d) einen oder mehrere Fettalkohol(e) mit einer gesättigten oder ungesättigten, verzweigten oder linearen $C_{12}$- bis $C_{22}$-Alkylkette,
wobei die Gesamtkonzentration der Verbindungen gemäß a) liegt im Bereich von 0,1 bis weniger als 5 Gew.-%, berechnet auf das Gesamtgewicht der Zusammensetzung, und

wobei das Gewichtsverhältnis der Verbindungen a) zu (b) + c)) im Bereich von 0,075 bis 8 liegt.

2. Zusammensetzung gemäß Anspruch 1, **dadurch gekennzeichnet, dass** die Gesamtkonzentration von Verbindungen gemäß b) im Bereich von 0,1 bis 10 Gew.-% liegt, vorzugsweise im Bereich von 0,2 bis 7,5 Gew.-%, mehr bevorzugt im Bereich von 0,5 bis 5 Gew.-%, ferner mehr bevorzugt im Bereich von 0,5 bis 2,5 Gew.-%, noch ferner mehr bevorzugt im Bereich von 1 bis 2,5 Gew.-%, berechnet auf das Gesamtgewicht der Zusammensetzung.

3. Zusammensetzung gemäß Ansprüchen 1 und/oder 2, **dadurch gekennzeichnet, dass** das Gewichtsverhältnis der Verbindungen a) zu (b) + c)) im Bereich von 0,15 bis 3, vorzugsweise im Bereich von 0,3 bis 2 liegt.

4. Zusammensetzung gemäß mindestens einem der vorhergehenden Ansprüche, **dadurch gekennzeichnet, dass** die Verbindung nach a) die folgende allgemeine Struktur aufweist

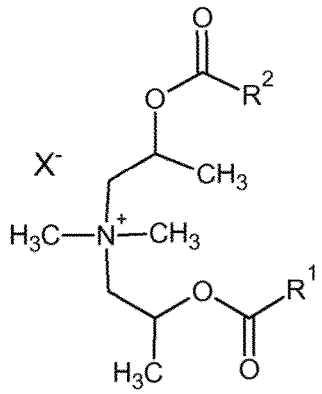

mit der Maßgabe, dass $R^1$ und $R^2$ sind $C_{11}$-$C_{21}$ lineare oder verzweigte, gesättigte oder ungesättigte Kohlenwasserstoffgruppen, die gleich oder verschieden sein können und gegebenenfalls mit einem oder mehreren Substituenten, ausgewählt aus Halogen, Hydroxyl, Amino und $C_1$-$C_4$-Alkoxy, substituiert sein können, und $X^-$ ist ausgewählt aus $Cl^-$, $Br^-$, $I^-$, Sulfat und Methosulfat.

5. Zusammensetzung gemäß mindestens einem der vorhergehenden Ansprüche, **dadurch gekennzeichnet, dass** die Verbindung nach a) Bis-(isostearoyl/oleoyl-isopropyl)-dimonium und/oder dessen Salz(e) ist.

6. Zusammensetzung gemäß mindestens einem der vorhergehenden Ansprüche, **dadurch gekennzeichnet, dass** die Gesamtkonzentration der Verbindungen nach d) im Bereich von 0,2 bis 10 Gew.-% liegt, vorzugsweise im Bereich von 0,5 bis 8 Gew.-%, ferner mehr bevorzugt im Bereich von 1 bis 6 Gew.-%, noch ferner mehr bevorzugt im Bereich von 2 bis 6 Gew.-%, berechnet auf das Gesamtgewicht der Zusammensetzung.

7. Zusammensetzung gemäß mindestens einem der vorhergehenden Ansprüche, **dadurch gekennzeichnet, dass** die Verbindungen nach d) ausgewählt sind aus Myristylalkohol, Cetylalkohol und Stearylalkohol und/oder deren Mischungen.

8. Zusammensetzung gemäß mindestens einem der vorhergehenden Ansprüche, **dadurch gekennzeichnet, dass** die Gesamtkonzentration der Verbindungen nach c) im Bereich von 0,05 bis 5 Gew.-% liegt, mehr bevorzugt im Bereich von 0,1 bis 2 Gew.-%, ferner mehr bevorzugt im Bereich von 0,25 bis 1,5 Gew.-%, berechnet auf das Gesamtgewicht der Zusammensetzung.

9. Zusammensetzung gemäß mindestens einem der vorhergehenden Ansprüche, **dadurch gekennzeichnet, dass** das Gewichtsverhältnis von Verbindung b) zu c) im Bereich von 5 bis 0,2 liegt.

10. Zusammensetzung gemäß mindestens einem der vorhergehenden Ansprüche, **dadurch gekennzeichnet, dass** die Verbindung(en) nach c) wässrige Emulsionen von aminierten Silikonen ist/sind.

11. Zusammensetzung gemäß mindestens einem der vorhergehenden Ansprüche, **dadurch gekennzeichnet, dass** die Zusammensetzung ein oder mehrere von a) verschiedene(s) Tensid(e) umfasst, vorzugsweise ausgewählt aus kationischen Tensiden und/oder nichtionischen Tensiden.

12. Zusammensetzung gemäß mindestens einem der vorhergehenden Ansprüche, **dadurch gekennzeichnet, dass** der pH-Wert der Zusammensetzung im Bereich von 3 bis 8 liegt, vorzugsweise im Bereich von 3,5 bis 7, noch bevorzugter im Bereich von 3,5 bis 4,5.

13. Verfahren zur Verringerung der Trocknungszeit von festen Substraten, vorzugsweise von Keratinsubstraten, besonders bevorzugt von menschlichen Keratinsubstraten, ferner besonders bevorzugt von menschlichem Haar, umfassend die Schritte:

a) Inkontaktbringen des festen Substrats mit einer wässrigen Zusammensetzung,
b) Auftragen der Zusammensetzung gemäß mindestens einem der Ansprüche 1 bis 12 auf das Substrat,
c) Belassen der Zusammensetzung auf dem Substrat für eine Zeitspanne von 10 s bis 600 s,
d) Abspülen des festen Substrats mit Wasser,
e) Belassen des festen Substrats, damit das Wasser abfließen kann,
f) gegebenenfalls Trocknen des Substrats mit einer Heizvorrichtung, die eine Lufttemperatur im Bereich von 35°C bis 230°C liefert.

14. Verfahren gemäß Anspruch 13, **dadurch gekennzeichnet, dass** zwischen den Schritten d) und f) keine Handtuchtrocknung durchgeführt wird.

15. Verwendung einer Zusammensetzung gemäß einem der Ansprüche 1 bis 12 zur Verbesserung des Wasserabflusses von festen Substraten.

**Revendications**

1. Composition pour réduire le temps de séchage de substrats solides, préférablement de substrats de kératine, plus préférentiellement de substrats de kératine humaine, encore plus préférablement de cheveux humains, comprenant :

a) un ou plusieurs tensioactif(s) d'ammonium quaternaire selon la structure générale suivante

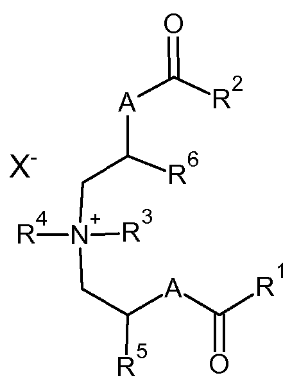

à la condition que $R^1$ et $R^2$ soient des groupes hydrocarbonés saturés ou insaturés, linéaires ou ramifiés, en $C_{12}$-$C_{21}$, qui peuvent être les mêmes ou différents et peuvent être facultativement substitués par un ou plusieurs substituants sélectionnés parmi un halogène, un hydroxyle, un amino, et un alcoxy en $C_1$-$C_4$,
$R^3$ et $R^4$ soient des groupes hydrocarbonés saturés ou insaturés, linéaires ou ramifiés, en $C_1$-$C_6$, qui peuvent être les mêmes ou différents et peuvent être facultativement substitués par un ou plusieurs substituants sélectionnés parmi un halogène, un hydroxyle, un amino, et
$R^5$ et $R^6$ soient H, des groupes hydrocarbonés saturés ou insaturés, linéaires ou ramifiés, en $C_1$-$C_{12}$, qui peuvent être les mêmes ou différents et les groupes hydrocarbonés peuvent être facultativement substitués par un ou plusieurs substituants sélectionnés parmi un halogène, un hydroxyle, un amino, et un alcoxy en $C_1$-$C_4$, et
A soit sélectionné parmi O et N, et $X^-$ soit sélectionné parmi $Cl^-$, $Br^-$, $I^-$, un sulfate, et un méthosulfate,

b) un ou plusieurs diméthylpolysiloxane(s) présentant un poids moléculaire moyen en poids $M_w$ dans la plage de 190 g/mol à 6 000 g/mol, déterminé par chromatographie par perméation de gel,
c) un ou plusieurs silicones aminés,

d) un ou plusieurs alcools gras présentant une chaîne alkyle en $C_{12}$ à $C_{22}$ saturée ou insaturée, ramifiée ou linéaire,
dans laquelle la concentration totale de composés selon a) est dans la plage de 0,1 % à moins de 5 % en poids, calculée par rapport au poids total de la composition, et
dans laquelle le rapport de poids des composés a) à (b) + c)) est dans la plage de 0,075 à 8.

**2.** Composition selon la revendication 1 **caractérisée en ce que** la concentration totale de composés selon b) est dans la plage de 0,1 % à 10 % en poids, préférablement dans la plage de 0,2 % à 7,5 % en poids, plus préférablement dans la plage de 0,5 % à 5 % en poids, encore plus préférablement dans la plage de 0,5 % à 2,5 % en poids, encore plus préférablement dans la plage de 1 % à 2,5 % en poids, calculée par rapport au poids total de la composition.

**3.** Composition selon les revendications 1 et/ou 2 **caractérisée en ce que** le rapport de poids des composés a) à (b) + c)) est dans la plage de 0,15 à 3, préférablement dans la plage de 0,3 à 2.

**4.** Composition selon l'une quelconque des revendications précédentes **caractérisée en ce que** le composé selon a) présente la structure générale suivante

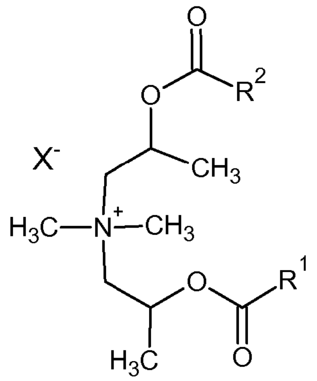

à la condition que $R^1$ et $R^2$ soient des groupes hydrocarbonés saturés ou insaturés, linéaires ou ramifiés, en $C_{11}$-$C_{21}$, qui peuvent être les mêmes ou différents et peuvent être facultativement substitués par un ou plusieurs substituants sélectionnés parmi un halogène, un hydroxyle, un amino, et un alcoxy en $C_1$-$C_4$, et $X^-$ est sélectionné parmi $Cl^-$, $Br^-$, $I^-$, un sulfate, et un méthosulfate.

**5.** Composition selon l'une quelconque des revendications précédentes **caractérisée en ce que** le composé selon a) est le bis-(isostéaroyl/oléoyl isopropyl) dimonium et/ou son (ses) sel(s).

**6.** Composition selon l'une quelconque des revendications précédentes **caractérisée en ce que** la concentration totale de composés selon d) est dans la plage de 0,2 % à 10 % en poids, préférablement dans la plage de 0,5 % à 8 % en poids, encore plus préférablement dans la plage de 1 % à 6 % en poids, encore plus préférablement dans la plage de 2 % à 6 % en poids, calculée par rapport au poids total de la composition.

**7.** Composition selon l'une quelconque des revendications précédentes **caractérisée en ce que** les composés selon d) sont sélectionnés parmi un alcool myristylique, un alcool cétylique, et un alcool stéarylique, et/ou leurs mélanges.

**8.** Composition selon l'une quelconque des revendications précédentes **caractérisée en ce que** la concentration totale de composés selon c) est dans la plage de 0,05 % à 5 % en poids, plus préférablement dans la plage de 0,1 % à 2 % en poids, encore plus préférablement dans la plage de 0,25 % à 1,5 % en poids, calculée par rapport au poids total de la composition.

**9.** Composition selon l'une quelconque des revendications précédentes **caractérisée en ce que** le rapport de poids des composés b) à c) est dans la plage de 5 à 0,2.

**10.** Composition selon l'une quelconque des revendications précédentes **caractérisée en ce que** le(s) composé(s) selon c) est (sont) des émulsions aqueuses de silicones aminés.

**11.** Composition selon l'une quelconque des revendications précédentes **caractérisée en ce que** la composition comprend un ou plusieurs tensioactif(s) différent(s) de a), de préférence sélectionnés parmi des tensioactifs cationiques et/ou tensioactifs non ioniques.

**12.** Composition selon l'une quelconque des revendications précédentes **caractérisée en ce que** le pH de la composition est dans la plage de 3 à 8, préférablement dans la plage de 3,5 à 7, plus préférablement dans la plage de 3,5 à 4,5.

**13.** Procédé pour réduire le temps de séchage de substrats solides, préférablement de substrats de kératine, plus préférablement de substrats de kératine humaine, encore plus préférablement de cheveux humains, comprenant les étapes consistant à :

a) mettre en contact le substrat solide avec une composition aqueuse,
b) appliquer la composition selon l'une quelconque des revendications 1 à 12 sur le substrat,
c) laisser la composition sur le substrat pendant une période de temps de 10 s à 600 s,
d) rincer le substrat solide avec de l'eau,
e) laisser le substrat solide pour permettre une évacuation de l'eau,
f) sécher facultativement le substrat avec un dispositif de chauffage délivrant une température d'air dans la plage de 35 °C à 230 °C.

**14.** Procédé selon la revendication 13 **caractérisé en ce que**, entre les étapes d) et f), aucun séchage à la serviette n'est mis en oeuvre.

**15.** Utilisation d'une composition telle que définie dans les revendications 1 à 12 pour améliorer une évacuation de l'eau de substrats solides.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 3059900
- EP 3168251 A
- DE 102015222976
- WO 9505800 A